# EUROPEAN PATENT APPLICATION

(11) **EP 3 064 135 A1**
(43) Date of publication of application: **07.09.2016**
(21) Application number: 13896491.1
(22) Date of filing: 11.11.2013
(51) Int. Cl.: A61B 5/12, A61F 11/00, H04L 29/08

(54) **INTERACTIVE HEARING DIAGNOSIS AND TREATMENT SYSTEM BASED ON WIRELESS MOBILE COMMUNICATION PLATFORM**

(30) Priority: 31.10.2013 CN 201310529945
(71) Applicant: Jiangsu Betterlife Medical Co., Ltd, Jiangsu 215500 (CN)
(72) Inventor: ZHAO, Yong David, Jiangsu 215500 (CN); ZHAO, Jennifer Jinping, Jiangsu 215500 (CN); ZHAO, Bingbing, Jiangsu 215500 (CN); XU, Hongwei, Jiangsu 215500 (CN)
(74) Representative: Becker Kurig Straus
(86) International application number: PCT/CN2013/001364
(87) International publication number: WO 2015/061925

(57) **Abstract**

Disclosed is a device for hearing tests and auditory assessments, including: an all-digital audio and tone signal synthesis generator for converting a logic algorithm and a parameter into a digital signal via built-in software, and inputting the digital signal into a digital signal processor (DSP) or processing the same with a PC central processor (CPU) to generate and output a multi-modal audio and tone acoustic signal; a hearing test module, comprising one or more objective and/or subjective hearing test submodules, sharing corresponding stimulation or interference signals converted by signals input by the same all-digital audio and tone signal synthesis generator, in order to perform a hearing test on a patient; and a comprehensive auditory assessment module for performing comprehensive auditory assessment on the hearing test result output by each hearing test submodule.

## Description

### FIELD OF THE INVENTION

The present disclosure relates to the field of hearing diagnosis and treatment system, and more particularly to an interactive hearing diagnosis and treatment system based on a wireless mobile communication platform.

### BACKGROUND OF THE INVENTION

Tinnitus or cranial tinnitus defined by American Academy of Audiology is a medical term, which means that the auditory perception generated by non-external sounds, and the sounds felt in the ear (one or both) or cranial cavity in the absence of the external sounds. Tinnitus or cranial tinnitus is a hissing, roaring, screaming, tweeting or clicking in form, and can be intermittent or continuous with a single note or a multiple note, and a constant or changeable intensity.

According to the traditional way, the patients can wear the digital hearing aids and digital tinnitus therapeutic apparatus after the personalized hearing test, the tinnitus diagnosis and the programming and debugging performed by the professional technical personnel in professional service center, which has the disadvantages or weaknesses as below:
1. Generally more than two sets of expensive testing apparatuses and two technical persons are involved;
2. Patients are required to receive the diagnose and treatment in the hospital or professional service center;
3. It is inconvenient to adjust in time with the changes of hearing and tinnitus of patients;
4. An efficiency is low because only one patient can be treated at a time;
5. Service is expensive;
6. It's not easy to popularize in the remote area;
7. It's not for the modem network technology.

### SUMMARY OF THE INVENTION

The technical problem to be solved in the present disclosure is to provide an interactive hearing diagnosis and treatment system based on a wireless mobile communication platform, with which the remote hearing diagnosis and treatment on one or more patients can be carried out by one or more experts, or one or more patients can carry out the hearing diagnosis and treatment with self-perception, and the diagnosis and treatment result data can be transmitted to the cloud data center to be remotely analyzed and used by experts, or the experts can be remotely requested to help the hearing diagnosis and treatment, so that a great amount of time can be saved, and the diagnosis and treatment on the patient can be carried out by experts without going out, and the interactive hearing diagnosis and treatment system is particularly suitable for being popularized and applied to the patient inconvenient to see a doctor in a remote area.

To solve the technical problem above, one technical solution in the present disclosure is to provide an interactive hearing diagnosis and treatment system based on the wireless mobile communication platform, wherein the interactive hearing diagnosis and treatment system includes an intelligent hearing diagnosis and treatment instrument for patient, an intelligent hearing diagnosis and treatment instrument for expert and a cloud data center; one or more intelligent hearing diagnosis and treatment instruments for expert are controlled by one or more experts and are connected to the cloud data center through the wireless mobile communication platform, and the cloud data center is connected to one or more intelligent hearing diagnosis and treatment instruments for the patient used by one or more patients through the wireless mobile communication platform; an internal structure of the intelligent hearing diagnosis and treatment instrument for patient includes a sound playing module, a first hearing testing and diagnosis module, a hearing aids fitting recipe module, a first tinnitus testing and diagnosis module, a tinnitus treatment recipe module, a first wireless communication module and a first operating system module; the first wireless communication module is communicational connected with the hearing aids fitting recipe module, the tinnitus treatment recipe module and the first operating system module respectively; the first operating system module is communicational connected with the first hearing testing and diagnosis module and the tinnitus treatment recipe module respectively; the first hearing testing and diagnosis module and the tinnitus treatment recipe module are communicational connected with the sound playing module respectively; wherein the first wireless communication module is communicational connected with the cloud data center; an internal structure of the intelligent hearing diagnosis and treatment instrument for expert includes a complex sound generator module, a second hearing testing and diagnosis module, a hearing aids fitting module, a second tinnitus testing and diagnosis module, a tinnitus treatment and program module, a second wireless communication module and a second operation system module; the second wireless communication module is communicational connected with the second operation system module, the second operation system module is communicational connected with the complex sound generator module, the second hearing testing and diagnosis module, the hearing aids fitting module, the second tinnitus testing and diagnosis module and the tinnitus treatment and program module respectively, wherein the second wireless communication module is communicational connected with the cloud data center; the internal structure of the cloud data center includes a data processing module, a data storage module, an expert database module, an exchanging center module and a wireless controlling module, the wireless controlling module is communicational connected with the data processing module, the data storage module, the expert database module and the exchanging center module, respectively.

In a preferred embodiment of the present disclosure, one or more patients get the result data of the diagnosis and treatment after the hearing diagnosis and treatment with self-perception through one or more intelligent hearing diagnosis and treatment instruments for patient, the result data of the diagnosis and treatment is transmitted to the cloud data center through the wireless mobile communication platform, and is transmitted to the intelligent hearing diagnosis and treatment instrument for expert through the wireless mobile communication platform to remote analysis and use or remote diagnosis and treatment by expert.

In a preferred embodiment of the present disclosure, the wireless mobile communication platform is GPRS and/or Android.

In a preferred embodiment of the present disclosure, the wireless controlling module includes an intercommunication video and audio controlling system.

In a preferred embodiment of the present disclosure, the intelligent hearing diagnosis and treatment instrument for patient is a mobile service platform, which includes a display screen, and the main interface of the display screen includes a tinnitus treatment unit, a treatment protocol unit, a treatment record unit and a tinnitus report unit.

The tinnitus treatment unit is configured to set the volume in the left ear and the right ear and the overall volume, and output to the earphone for playing;

The treatment protocol unit is configured to set the length or the course of treatment, the choice and the mixing mode of the tinnitus treatment protocol and the sequence and the length of the treatment audio tones playing, those set can be made on either or both ears;

The treatment record unit is configured to record the adopted treatment protocol, the date, time and the length of treatment, or delete the treatment record;

The tinnitus report unit is configured to transform the testing report and the treatment report, display the doctor's advice, and extract the current report to the root directory of the memory card to save or print.

In a preferred embodiment of the present disclosure, the intelligent hearing diagnosis and treatment instrument for patient further includes one or more from the earphone, the printer, the hearing aid and the tinnitus treatment instrument.

In a preferred embodiment of the present disclosure, the earphone is a full frequency earphone with the frequency range between 0-20kHz.

In a preferred embodiment of the present disclosure, the intelligent hearing diagnosis and treatment instrument for expert is the mobile service platform including a displayer and a pillow. The main interface of the displayer includes a testing and fitting module and an operating system module, the testing and fitting module is located at the right end of the main interface, and the operating system module is at the left end.

In a preferred embodiment of the present disclosure, the testing and fitting module includes a hearing testing unit, a tinnitus testing unit, a tinnitus treatment unit, an ear canal examination unit and a hearing aid fitting unit.

The hearing testing unit is configured to output the audiogram through the air conduction or the bone conduction, and save it;

The tinnitus testing unit is configured to adopt one or more from these three methods such as the multichannel acoustic synthesis, the tone curve fitting and the background audio tones database to synthesize or compound a multi-modal tinnitus audio tone signals; the multichannel acoustic synthesis includes a plurality of frequency channels such as eight which are arranged to the frequency of right ear and left ear and the frequency channel, with the frequency range between 0-20kHz, the stepping equal or greater than 1Hz and no more than 10Hz, with the loudness range between0-120db, the stepping equal or greater than 1db and no more than 10db; the tone curve fitting includes the setting of wavenumber and waveform in given time; the background audio tones database is the material stored or downloaded from the cloud data center, and the parameters adjustment such as adjusting the frequency, loudness, the composition removing or adding is made after the background audio tones material is called, whereas the audio tones material generated by the tinnitus testing unit is uploaded to the cloud data center as the reference by others, thereby forming an interactive between the patients or the patients and the experts;

The tinnitus treatment unit is configured to generate the patient diagnosed cases and provide the masking therapy for patient.

The ear canal examination unit is configured to examine the ear canal and the eardrum appearance by using the ear canal digital image;

The hearing aid fitting unit is configured to output the corresponding patient fitting information according to the patient testing result, then choose the suitable hearing aid product for patient, at the same time according to the tinnitus diagnosis data, adjust the position or programs and debug the process of the tinnitus hearing aids or tinnitus treatment instruments accurately.

In a preferred embodiment of the present disclosure, the operating system module includes an adding unit, a file unit, a testing report unit, a treatment report unit, a client unit, a deletion unit and a logout unit.
The adding unit: adds the file information of patient and records the degree of the tinnitus;
The file unit: views or modifies the file information of patient;
The test report unit: generates various testing report templates on request;
The treatment report unit: generates a corresponding treatment report according to the testing report;
The deletion unit is configured to delete all kinds of data information;
The logout unit is configured to save and exit.

The beneficial effect of the present disclosure is: with the interactive hearing diagnosis and treatment system based on a wireless mobile communication platform in the present disclosure, the remote hearing diagnosis and treatment on one or more patients can be carried out by one or more experts, or one or more patients can carry out the hearing diagnosis and treatment with self-perception, and the diagnosis and treatment result data can be transmitted to the cloud data center to be remotely analyzed and used by experts. The efficiency of diagnosis and treatment for tinnitus indication is about 80%, so that a great amount of time can be saved, and the experts can diagnose and treat the patient without going out, and the interactive hearing diagnosis and treatment system is particularly suitable for being popularized and applied to the patient inconvenient to see a doctor in a remote area.

### BRIEF DESCRIPTION OF THE DRAWINGS

In order to clearly describe the technical solution, the embodiments of the present disclosure are described in detail below with reference to the following drawings. It is obvious that the following described drawings are only some of the embodiments of the invention, those skilled in the art may obtain other drawings according to these drawings without an inventive step.
FIG. 1 is a structure diagram showing an interactive hearing diagnosis and treatment system based on a wireless mobile communication platform in accordance with a preferred embodiment of the present disclosure.
FIG. 2 is an inside structure diagram of an intelligent hearing diagnosis and treatment instrument for patient of FIG. 1.
FIG 3 is an inside structure diagram of an intelligent hearing diagnosis and treatment instrument for expert of FIG. 1.
FIG. 4 is an inside structure diagram of a cloud data center of FIG. 1.
FIG. 5 is a schematic diagram of a main interface of an intelligent hearing diagnosis and treatment instrument for patient of FIG. 1.
FIG. 6 is a schematic diagram of a main interface of an intelligent hearing diagnosis and treatment instrument for expert of FIG. 1.

### DETAILED DESCRIPTION OF THE INVENTION

The technical solution of the embodiments of the present disclosure will be clearly and completely described. Obviously, the embodiments described herein are some embodiments of the present disclosure, but not all embodiments. Any other embodiments educed by those skilled in the art based on the embodiments described herein without any creative activities should fall into the protection scope of this invention.

Referring to FIG. I, the embodiments of the present disclosure include:
An interactive hearing diagnosis and treatment system based on a wireless mobile communication platform includes an intelligent hearing diagnosis and treatment instrument for patient, an intelligent hearing diagnosis and treatment instrument for expert and a cloud data center. One or more intelligent hearing diagnosis and treatment instruments for expert are controlled by one or more experts to be connected to the cloud data center through the wireless mobile communication platform, and the cloud data center is connected to one or more intelligent hearing diagnosis and treatment instruments for the patient used by one or more patients through the wireless mobile communication platform.

The wireless mobile communication platform is GPRS and/or Android. One expert can connect with more patients through the wireless mobile communication platform, and the hearing diagnosis and treatment instrument for expert at one end is connected with the hearing diagnosis and treatment instrument for patient at the other end through the cloud data center, thereby the remote control of the hearing diagnosis and treatment is carried out.

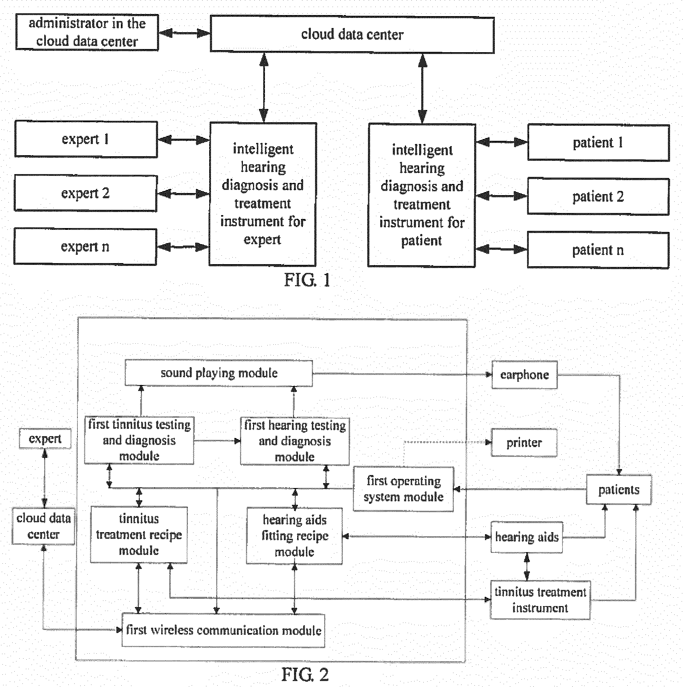

Further, as shown in FIG. 2, the internal structure of the intelligent hearing diagnosis and treatment instrument for patient includes a sound playing module, a first hearing testing and diagnosis module, a hearing aids fitting recipe module, a first tinnitus testing and diagnosis module, a tinnitus treatment recipe module, a first wireless communication module and a first operating system module. The first wireless communication module is communicational connected with the hearing aids fitting recipe module, the tinnitus treatment recipe module and the first operating system module respectively. The first operating system module is communicational connected with the first hearing testing and diagnosis module and the tinnitus treatment recipe module respectively. The first hearing testing and diagnosis module and the tinnitus treatment recipe module are communicational connected with the sound playing module respectively. The first wireless communication module is communicational connected with the cloud data center.

The intelligent hearing diagnosis and treatment instrument for patient further includes one or more from earphone, printer, hearing aid and tinnitus treatment instrument. The earphone is a full frequency earphone with the frequency range between 0-20kHz.

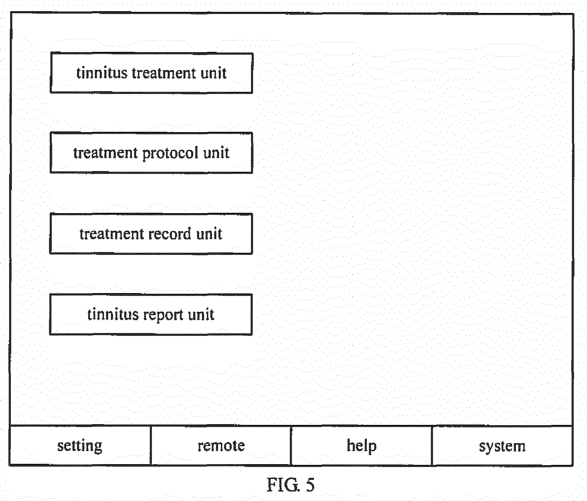

The intelligent hearing diagnosis and treatment instrument for patient is a portable, miniaturized and intelligent mobile service platform with a touch panel as the display screen. As shown in FIG. 5, the main interface of the display screen includes a tinnitus treatment unit, a treatment protocol unit, a treatment record unit and a tinnitus report unit.

The tinnitus treatment unit is configured to set the volume in the left ear and the right ear and the overall volume, and output to the earphone for playing;

The treatment protocol unit is configured to set the length or the course of treatment, the choice and the mixing mode of the tinnitus treatment protocol and the sequence and the length of the treatment audio tones playing, those set can be made on either or both ears;

The treatment record unit is configured to record the adopted treatment protocol, the date, time and the length of treatment, or delete the treatment record;

The tinnitus report unit is configured to transform the testing report and the treatment report, display the doctor's advice, and extract the current report to the root directory of the memory card to save or print.

Users click the tinnitus treatment unit on the main interface of the intelligent hearing diagnosis and treatment instrument for patient to enter. If the treatment protocols are not defined, users should choose certain treatment audio in the list, if the treatment protocols are defined, users do not need to choose. If there is no touch screen operation within 1 minute, the system will be transferred to a power save mode and the screen will darken, which can be activate by touching the screen again.

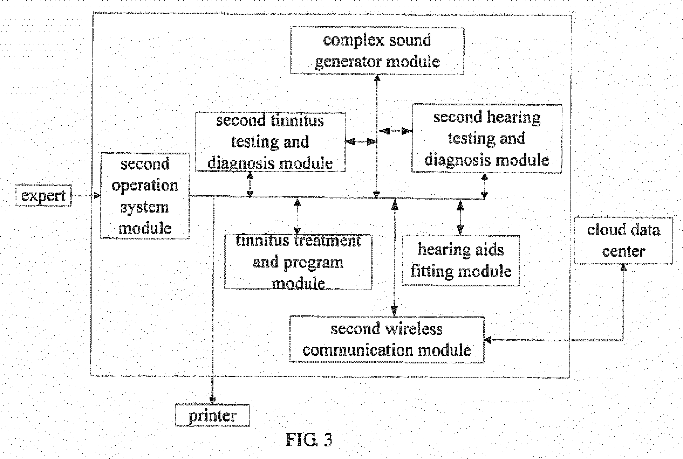

As shown in FIG. 3, the internal structure of the intelligent hearing diagnosis and treatment instrument for expert includes a complex sound generator module, a second hearing testing and diagnosis module, a hearing aids fitting module, a second tinnitus testing and diagnosis module, a tinnitus treatment and program module, a second wireless communication module and a second operation system module. The second wireless communication module is communicational connected with the second operation system module, the second operation system module is communicational connected with the complex sound generator module, the second hearing testing and diagnosis module, the hearing aids fitting module, the second tinnitus testing and diagnosis module and the tinnitus treatment and program module respectively, the second wireless communication module is communicational connected with the cloud data center.

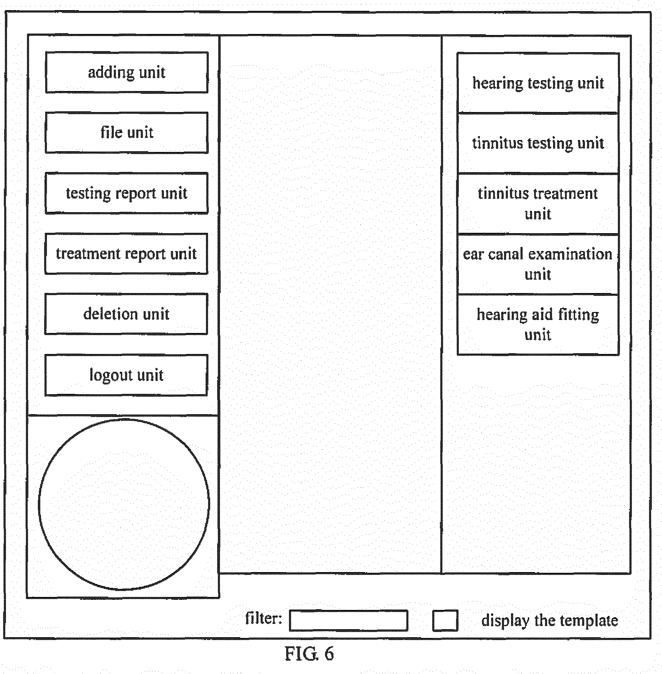

As shown in FIG. 6, the intelligent hearing diagnosis and treatment instrument for expert is the mobile service platform including a displayer and a pillow. The main interface of the displayer includes a testing and fitting module and an operating system module, the testing and fitting module is located at the right end of the main interface, and the operating system module is at the left end. Meanwhile, the interfacial design of the testing and fitting module and the operating system module may be switched or arranged at the upper and lower position.

The testing and fitting module includes a hearing testing unit, a tinnitus testing unit, a tinnitus treatment unit, an ear canal examination unit and a hearing aid fitting unit.

The hearing testing unit is configured to output the audiogram through the air conduction or the bone conduction, and save it;

The tinnitus testing unit is configured to adopt one or more from these three methods such as the multichannel acoustic synthesis, the tone curve fitting and the background audio tones database to synthesize or compound a multi-modal tinnitus audio tone signals; the multichannel acoustic synthesis includes a plurality of frequency channels such as eight which are arranged to the frequency of right ear and left ear and the frequency channel, with the frequency range between 0-20kHz, the stepping equal or greater than 1Hz and no more than 10Hz, with the loudness range between0-120db, the stepping equal or greater than 1db and no more than 10db; the tone curve fitting includes the setting of wavenumber and waveform in given time; the background audio tones database is the material stored or downloaded from the cloud data center, and the parameters adjustment such as adjusting the frequency, loudness, the composition removing or adding is made after the background audio tones material is called, whereas the audio tones material generated by the tinnitus testing unit is uploaded to the cloud data center as the reference by others, thereby forming an interactive between the patients or the patients and the experts;

The tinnitus treatment unit is configured to generate the patient diagnosed cases and provide the masking therapy for patient.

The ear canal examination unit is configured to examine the ear canal and the eardrum appearance by using the ear canal digital image;

The hearing aid fitting unit: outputs the patient fitting information according to the patient testing result, then chooses the suitable hearing aid product for patient, at the same time according to the tinnitus diagnosis data, adjusts the position or programs and debugs the process of the tinnitus hearing aids or tinnitus treatment instruments accurately.

The operating system module includes an adding unit, a file unit, a testing report unit, a treatment report unit, a client unit, a deletion unit and a logout unit.
The adding unit: adds the file information of patient and records the degree of the tinnitus;
The file unit: views or modifies the file information of patient;
The test report unit: generates various testing report templates on request;
The treatment report unit: generates a corresponding treatment report according to the testing report;
The deletion unit is configured to delete all kinds of data information;
The logout unit is configured to save and exit.

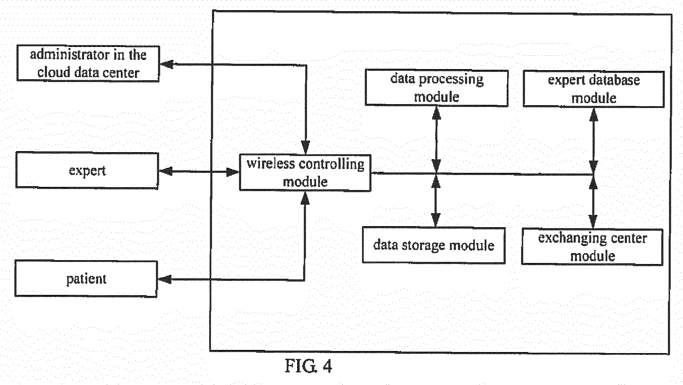

As shown in FIG. 4, the internal structure of the cloud data center includes a data processing module, a data storage module, an expert database module, an exchanging center module and a wireless controlling module, the wireless controlling module is communicational connected with the data processing module, the data storage module, the expert database module and the exchanging center module respectively. The wireless controlling module includes an intercommunication video and audio controlling system.

Several experts and patients can connect with the cloud data center individually and control the hearing diagnosis and treatment interactively and remotely through the wireless controlling module; the administer of the cloud data center is in charge of maintenance of the running of cloud data center, technical support, classification and storage of the data such as copying the typical data in the data storage module to the expert database module, accumulating the typical customers' data and expanding the expert database module for public use.

The data processing module: the real-time data interchanging, conversion and transmission;

The data storage module: the database of patients and experts stored in category;

The expert database module: to collect and store the hearing compensating recipe of the typical cases, all kinds of tinnitus modal data and the corresponding treatment prescriptions of tinnitus masking and sound conditioning, stored in category;

The exchanging center module: the multi-exchange of technology, data, experience or chatting between experts and patients, especially to build a patients group where the patients with similar to hearing loss or tinnitus communicate and help with each other, which can help to improve the hearing diagnosis and treatment efficiency.

Further, according to the interactive hearing diagnosis and treatment system based on the wireless mobile communication platform of the present disclosure, patients can manage or adjust several treatment protocols with self perception, or complete such management or adjustment of treatment protocols under the guidance of remote control by experts. The result data of the diagnosis and treatment can be made after one or more patients carry out the hearing diagnosis and treatment with self-perception through one or more intelligent hearing diagnosis and treatment instruments for patient, the result data of the diagnosis and treatment is transmitted to the cloud data center through the wireless mobile communication platform, and is transmitted to the intelligent hearing diagnosis and treatment instrument for expert through the wireless mobile communication platform to remote analysis and use or remote diagnosis and treatment by expert.

The debugging or remote controlling of the hearing diagnosis and hearing aids fitting with self perception: patients can start the calibrated audiometer embedded in the hearing testing and diagnosis module, perceive and hear the audio signal directly through the sound playing module and earphone, record the hearing threshold under the different frequencies through the operating system module, and the audiogram representing the hearing loss thereof can be automatically generated and transmitted to the cloud data center. After remote receiving the audiogram, experts carry out the hearing aids programming and fitting on the intelligent hearing diagnosis and treatment instrument for expert, and send the fitting results or recipes to the intelligent hearing diagnosis and treatment instrument for patient and the connected hearing aids; patients can connect the audiogram to the hearing aids fitting recipe module by themselves, automatically fit and debug the hearing aids they have; the hearing diagnosis can also be wireless connected between experts and patients, and experts control the hearing testing and diagnosis module in their own intelligent hearing diagnosis and treatment instruments for expert, transmit the audio signals to intelligent hearing diagnosis and treatment instruments for patient and earphones which can be perceived by patients, and the personalized audiograms are generated after the interactive between experts and patients.

The debugging or remote controlling of the tinnitus diagnosis and treatment with self perception: patients start the calibrated multi-modal tinnitus diagnosis instrument embedded in the hearing testing and diagnosis module, perceive and hear the audio tones signal directly through the sound playing module and earphone, confirm the tinnitus modal is similar or the same, and perceive and hear the audio tones signal directly through the sound playing module and earphone, record the selected tinnitus mode that is best-fit for their own tinnitus conditions through the operating system module, then and treatment prescriptions of tinnitus masking and sound conditioning can be automatically generated by the tinnitus treatment recipe module; the other way is that patients wireless connect to the cloud data center and experts directly, and experts control the tinnitus testing and diagnosis module in the intelligent hearing diagnosis and treatment instrument for expert and remotely transmit the selected tinnitus modal to patients' ears, then patients perceive and give feedback to experts, thereby one or more suitable tinnitus modal can be confirmed after several interactions between patients and experts, and input the tinnitus treatment recipe in the intelligent hearing diagnosis and treatment instrument for patient, automatically generate one or more treatment prescriptions of tinnitus masking and sound conditioning, then input in the tinnitus treatment instruments patients have.

For patients with both hearing loss and tinnitus, those two steps can be combined that input the tinnitus prescriptions to the hearing aids.

Due to the changes in hearing loss or tinnitus, experts or patients both can set several courses of hearing treatment recipe or tinnitus treatment recipe according to individual conditions, patients perceive and select, and can mix and fit several recipes to get a new treatment recipe.

With the interactive hearing diagnosis and treatment system based on a wireless mobile communication platform in the present disclosure, one or more experts can help one or more patients to carry out the remote hearing diagnosis and treatment, and one or more patients can carry out the hearing diagnosis and treatment with self-perception, and transmit the diagnosis and treatment result data to the cloud data center to be remotely analyzed and used by experts, or remotely request experts to help the hearing diagnosis and treatment. The fully automatically medical informatization management is realized by the seamless connection between various segments through the wireless mobile communication platform, so that a great amount of time can be saved, and the diagnosis and treatment on the patient can be carried out by experts without going out, and the interactive hearing diagnosis and treatment system is particularly suitable for being popularized and applied to the patient inconvenient to see a doctor in a remote area.

Although particular embodiments of the invention have been described as above, it will be understood that they are not intended to limit the patent scope of the present disclosure, and it will be obvious that the equivalent structural changes or process transformation based on the description and drawings of the present disclosure should be covered within the scope of the present disclosure, as well as the embodiments applied in the other relevant technical field directly or indirectly.

## Claims

1. An interactive hearing diagnosis and treatment system based on the wireless mobile communication platform, wherein the interactive hearing diagnosis and treatment system comprises an intelligent hearing diagnosis and treatment instrument for patient, an intelligent hearing diagnosis and treatment instrument for expert and a cloud data center; one or more intelligent hearing diagnosis and treatment instruments for expert are controlled by one or more experts and are connected to the cloud data center through the wireless mobile communication platform, and the cloud data center is connected to one or more intelligent hearing diagnosis and treatment instruments for the patient used by one or more patients through the wireless mobile communication platform; an internal structure of the intelligent hearing diagnosis and treatment instrument for patient comprises a sound playing module, a first hearing testing and diagnosis module, a hearing aids fitting recipe module, a first tinnitus testing and diagnosis module, a tinnitus treatment recipe module, a first wireless communication module and a first operating system module; the first wireless communication module is communicational connected with the hearing aids fitting recipe module, the tinnitus treatment recipe module and the first operating system module respectively; the first operating system module is communicational connected with the first hearing testing and diagnosis module and the tinnitus treatment recipe module respectively; the first hearing testing and diagnosis module and the tinnitus treatment recipe module are communicational connected with the sound playing module respectively; wherein the first wireless communication module is communicational connected with the cloud data center; an internal structure of the intelligent hearing diagnosis and treatment instrument for expert comprises a complex sound generator module, a second hearing testing and diagnosis module, a hearing aids fitting module, a second tinnitus testing and diagnosis module, a tinnitus treatment and program module, a second wireless communication module and a second operation system module; the second wireless communication module is communicational connected with the second operation system module, the second operation system module is communicational connected with the complex sound generator module, the second hearing testing and diagnosis module, the hearing aids fitting module, the second tinnitus testing and diagnosis module and the tinnitus treatment and program module respectively, wherein the second wireless communication module is communicational connected with the cloud data center; the internal structure of the cloud data center comprises a data processing module, a data storage module, an expert database module, an exchanging center module and a wireless controlling module, the wireless controlling module is communicational connected with the data processing module, the data storage module, the expert database module and the exchanging center module, respectively.

2. The interactive hearing diagnosis and treatment system based on the wireless mobile communication platform of claim 1, wherein one or more patients get a result data of the diagnosis and treatment after the hearing diagnosis and treatment with self-perception through one or more intelligent hearing diagnosis and treatment instruments for patient, the result data of the diagnosis and treatment is transmitted to the cloud data center through the wireless mobile communication platform, and is transmitted to the intelligent hearing diagnosis and treatment instrument for expert through the wireless mobile communication platform for the remote analysis and use or remote diagnosis and treatment by expert.

3. The interactive hearing diagnosis and treatment system based on the wireless mobile communication platform of claim 1 or 2, wherein the wireless mobile communication platform is GPRS and/or Android.

4. The interactive hearing diagnosis and treatment system based on the wireless mobile communication platform of claim 1, wherein the wireless controlling module comprises an intercommunication video and audio controlling system.

5. The interactive hearing diagnosis and treatment system based on the wireless mobile communication platform of claim 1, wherein the intelligent hearing diagnosis and treatment instrument for patient is a mobile service platform, which comprises a display screen, and the main interface of the display screen comprises a tinnitus treatment unit, a treatment protocol unit, a treatment record unit and a tinnitus report unit,
the tinnitus treatment unit is configure to set the volume in the left ear and the right ear and the overall volume, and output to the earphone for playing;
the treatment protocol unit is configure to set the length or the course of treatment, the choice and the mixing mode of the tinnitus treatment protocol and the sequence and the length of the treatment audio tones playing, those set can be made on either or both ears;
the treatment record unit is configure to record the adopted treatment protocol, the date, time and the length of treatment, or delete the treatment record;
the tinnitus report unit is configure to transform the testing report and the treatment report, display the doctor's advice, and extract the current report to the root directory of the memory card to save or print.

6. The interactive hearing diagnosis and treatment system based on the wireless mobile communication platform of claim 1, wherein the intelligent hearing diagnosis and treatment instrument for patient further comprises one or more from the earphone, the printer, the hearing aid and the tinnitus treatment instrument.

7. The interactive hearing diagnosis and treatment system based on the wireless mobile communication platform of claim 6, wherein the earphone is a full frequency earphone with the frequency range between 0-20kHz.

8. The interactive hearing diagnosis and treatment system based on the wireless mobile communication platform of claim 1, wherein the intelligent hearing diagnosis and treatment instrument for expert is the mobile service platform comprising a displayer and a pillow. The main interface of the displayer comprises a testing and fitting module and an operating system module, the testing and fitting module is located at the right end of the main interface, and the operating system module is at the left end.

9. The interactive hearing diagnosis and treatment system based on the wireless mobile communication platform of claim 8, wherein the testing and fitting module comprises a hearing testing unit, a tinnitus testing unit, a tinnitus treatment unit, an ear canal examination unit and a hearing aid fitting unit,
the hearing testing unit is configure to output the audiogram through the air conduction or the bone conduction, and save it;
the tinnitus testing unit is configured to adopt one or more from these three methods such as the multichannel acoustic synthesis, the tone curve fitting and the background audio tones database to synthesize or compound a multi-modal tinnitus audio tone signals; the multichannel acoustic synthesis comprises a plurality of frequency channels such as eight which are arranged to the frequency of right ear and left ear and the frequency channel, with the frequency range between 0-20kHz, the stepping equal or greater than 1Hz and no more than 10Hz, with the loudness range between0-120db, the stepping equal or greater than 1db and no more than 10db; the tone curve fitting comprises the setting of wavenumber and waveform in given time; the background audio tones database is the material stored or downloaded from the cloud data center, and the parameters adjustment such as adjusting the frequency, loudness, the composition removing or adding is made after the background audio tones material is called, whereas the audio tones material generated by the tinnitus testing unit is uploaded to the cloud data center as the reference by others, thereby forming an interactive between the patients or the patients and the experts;
the tinnitus treatment unit is configured to generate the patient diagnosed cases and provide the masking therapy for patient,
the ear canal examination unit is configured to examine the ear canal and the eardrum appearance by using the ear canal digital image;
the hearing aid fitting unit is configured to output the corresponding patient fitting information according to the patient testing result, then choose the suitable hearing aid product for patient, at the same time according to the tinnitus diagnosis data, adjust the position or programs and debug the process of the tinnitus hearing aids or tinnitus treatment instruments accurately.

10. The interactive hearing diagnosis and treatment system based on the wireless mobile communication platform of claim 8, wherein the operating system module comprises an adding unit, a file unit, a testing report unit, a treatment report unit, a client unit, a deletion unit and a logout unit,
the adding unit is configured to add the file information of patient and record the degree of the tinnitus;
the file unit is configured to view or modify the file information of patient;
the test report unit is configured to generate various testing report templates on request;
the treatment report unit is configured to generate a corresponding treatment report according to the testing report;
the deletion unit is configured to delete all kinds of data information; the logout unit is configured to save and exit.
